# EUROPEAN PATENT APPLICATION

(11) **EP 2 486 937 A1**
(43) Date of publication of application: **15.08.2012**
(21) Application number: 12167479.0
(22) Date of filing: 24.05.2007
(51) Int. Cl.: A61K 38/48, A61K 9/00, A61L 31/00, A61P 13/00

(54) **Methods for treating a urethral stricture with a botulinum toxin**

(30) Priority: 02.06.2006 US 446403
(62) Divisional of application: 07797720.5
(71) Applicant: ALLERGAN, INC., Irvine CA 92612 (US)
(72) Inventor: Brooks, Gregory F., Irvine, CA, 92612 (US); Donovan, Stephen, Capistrano Beach, CA, 92624 (US)
(74) Representative: HOFFMANN EITLE

(57) **Abstract**

Methods of treating a urethral stricture in a patient. The methods can include the step of administering a therapeutically effective amount of a botulinum toxin directly to a urethra of the patient, for example by use of a stent from which the botulinum toxin can elute or diffuse, thereby treating the urethra stricture.

## Description

### BACKGROUND

The present invention relates to methods for treating a urethral stricture in a mammal, such as a human patient. In particular, the present invention is directed to methods for treating a urethral stricture using a stent to which a botulinum toxin has been associated, as by coating or embedding the stent with the botulinum toxin.

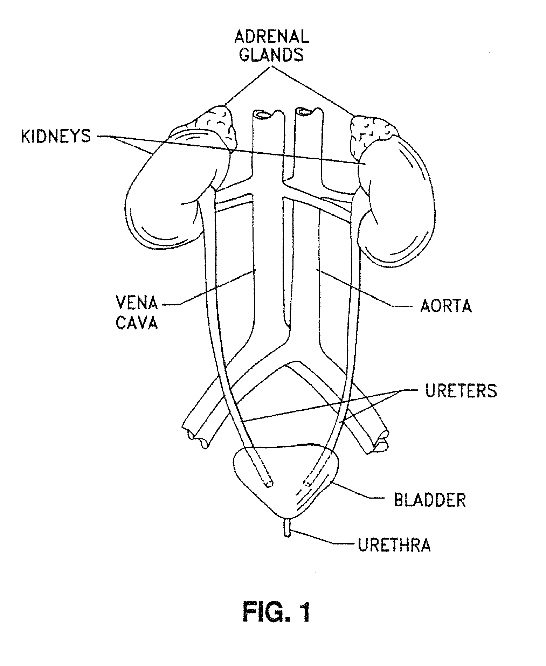

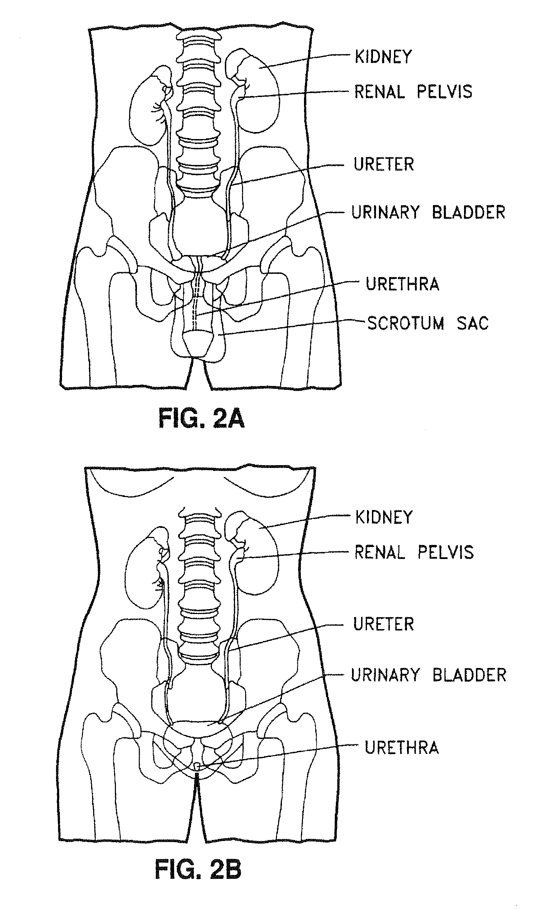

A urethra is a tube or vessel which carries urine from the bladder to the exterior of the body (Figure 1). The urethra tube is typically longer in men than the urethra tube is in women (see Figures 2a and 2b, respectively).

A urethral stricture is an abnormal narrowing of the non-muscular tissue of the urethra (see Figure 1). A urethral stricture can be caused by an inflammation, ischemia, or trauma at the urethra site. For example, an inflammation, ischemia, or trauma at the urethra site can cause a scar tissue therein. Once formed, the scar tissue contracts and causes the urethral stricture. A urethral stricture is not due to a spasm of a urethral muscle, such as in the case of the muscle spasm in a detrusor sphincter. Acquired urethral stricture is a common problem in male patients in which the urethra is narrowed, usually from infection or trauma. Pathologically, the urethral opening becomes narrowed by a buildup of fibrotic tissue. The resulting stricture of the urethral opening hampers the excretion of urine.

The term urethral stricture generally refers to the anterior urethra and is secondary to scarring in the spongy erectile tissue of the corpus spongiosum. A posterior urethral stricture is due to a fibrotic process that narrows the bladder neck and usually results from a distraction injury secondary to trauma or surgery, such as radical prostatectomy.

A common technique for treating a urethral stricture is dilation of the urethra, for example by insertion of a urethral stent. Stenting is a procedure in which a cylindrical structure (stent) is placed into a hollow tubular organ, e.g., the urethra, to provide artificial support and maintain the patency of the opening. However, one of the problems associated with this technique is that the dilation process may cause additional scarring, which may eventually worsen the stricture.

An alternative technique for treating a urethral stricture is an internal urethrotomy, which involves incising the stricture transurethrally. The incision allows release of the scar tissue. However, there are frequent complications with this technique. For example, the complications include recurrence of stricture, bleeding, extravasation of irrigation fluid into perispongial tissues and increasing fibrotic response.

The success rate for the treatment of a urethral stricture with the traditional techniques is not very good, due to the above mentioned complications. Therefore, there exists a need for an improved method for treating a urethral stricture.

### Botulinum toxin

The anaerobic, Gram positive bacterium Clostridium botulinum produces a potent polypeptide neurotoxin, Botulinum toxin, which causes a neuroparalytic illness in humans and animals referred to as botulism. The spores of Clostridium botulinum are found in soil and can grow in improperly sterilized and sealed food containers of home based canneries, which are the cause of many of the cases of botulism. The effects of botulism typically appear 18 to 36 hours after eating the foodstuffs infected with a Clostridium botulinum culture or spores. The botulinum toxin can apparently pass unattenuated through the lining of the gut and attack peripheral motor neurons. Symptoms of botulinum toxin intoxication can progress from difficulty walking, swallowing, and speaking to paralysis of the respiratory muscles and death.

Botulinum toxin type A ("BoNT/A") is the most lethal natural biological agent known to man. About 50 picograms of Botulinum toxin (purified neurotoxin complex) serotype A is a LD₅₀ in mice. One unit (U) of botulinum toxin is defined as the LD₅₀ upon intraperitoneal injection into female Swiss Webster mice weighing 18-20 grams each. Seven immunologically distinct botulinum neurotoxins have been characterized, these being respectively botulinum neurotoxin serotypes A, B, C₁, D, E, F and G each of which is distinguished by neutralization with serotype-specific antibodies. The different serotypes of botulinum toxin vary in the animal species that they affect and in the severity and duration of the paralysis they evoke. For example, it has been determined that BoNt/A is 500 times more potent, as measured by the rate of paralysis produced in the rat, than is botulinum toxin serotype B (BoNT/B). Additionally, botulinum toxin type B ("BoNt/B") has been determined to be non-toxic in primates at a dose of 480 U/kg which is about 12 times the primate LD₅₀ for BoNt/A. Botulinum toxin apparently binds with high affinity to cholinergic motor neurons, is translocated into the neuron and blocks the release of acetylcholine.

Botulinum toxins have been used in clinical settings for the treatment of neuromuscular disorders characterized by hyperactive skeletal muscles. BoNt/A has been approved by the U.S. Food and Drug Administration for the treatment of blepharospasm, strabismus, hemifacial spasm and cervical dystonia. Additionally, a botulinum toxin type B has been approved by the FDA for the treatment of cervical dystonia. Non-serotype A botulinum toxin serotypes apparently have a lower potency and/or a shorter duration of activity as compared to BoNt/A. Clinical effects of peripheral intramuscular BoNt/A are usually seen within one week of injection. The typical duration of symptomatic relief from a single intramuscular injection of BoNt/A averages about three months.

Although all the botulinum toxins serotypes apparently inhibit release of the neurotransmitter acetylcholine at the neuromuscular junction, they do so by affecting different neurosecretory proteins and/or cleaving these proteins at different sites. For example, botulinum serotypes A and E both cleave the 25 kiloDalton (kD) synaptosomal associated protein (SNAP-25), but they target different amino acid sequences within this protein. BoNT/B, D, F and G act on vesicle-associated protein (VAMP, also called synaptobrevin), with each serotype cleaving the protein at a different site. Finally, botulinum toxin serotype C₁ (BoNTlC₁) has been shown to cleave both syntaxin and SNAP-25. These differences in mechanism of action may affect the relative potency and/or duration of action of the various botulinum toxin serotypes.

Regardless of serotype, the molecular mechanism of toxin intoxication appears to be similar and to involve at least three steps or stages. In the first step of the process, the toxin binds to the presynaptic membrane of the target neuron through a specific interaction between the H chain and a cell surface receptor; the receptor is thought to be different for each serotype of botulinum toxin and for tetanus toxin. The carboxyl end segment of the H chain, H_{C}, appears to be important for targeting of the toxin to the cell surface.

In the second step, the toxin crosses the plasma membrane of the poisoned cell. The toxin is first engulfed by the cell through receptor-mediated endocytosis, and an endosome containing the toxin is formed. The toxin then escapes the endosome into the cytoplasm of the cell. This last step is thought to be mediated by the amino end segment of the H chain, H_{N}, which triggers a conformational change of the toxin in response to a pH of about 5.5 or lower. Endosomes are known to possess a proton pump which decreases intra endosomal pH. The conformational shift exposes hydrophobic residues in the toxin, which permits the toxin to embed itself in the endosomal membrane. The toxin then translocates through the endosomal membrane into the cytosol.

The last step of the mechanism of botulinum toxin activity appears to involve reduction of the disulfide bond joining the H and L chain. The entire toxic activity of botulinum and tetanus toxins is contained in the L chain of the holotoxin; the L chain is a zinc (Zn++) endopeptidase which selectively cleaves proteins essential for recognition and docking of neurotransmitter-containing vesicles with the cytoplasmic surface of the plasma membrane, and fusion of the vesicles with the plasma membrane. Tetanus neurotoxin, botulinum toxin/B/D,/F, and/G cause degradation of synaptobrevin (also called vesicle-associated membrane protein (VAMP)), a synaptosomal membrane protein. Most of the VAMP present at the cytosolic surface of the synaptic vesicle is removed as a result of any one of these cleavage events. Each toxin specifically cleaves a different bond.

The molecular weight of the botulinum toxin protein molecule, for all seven of the known botulinum toxin serotypes, is about 150 kD. lnterestingly, the botulinum toxins are released by Clostridial bacterium as complexes comprising the 150 kD botulinum toxin protein molecule along with associated non-toxin proteins. Thus, the BoNt/A complex can be produced by Clostridial bacterium as 900 kD, 500 kD and 300 kD forms. BoNT/ B and C₁ are apparently produced as only a 500 kD complex. BoNT/D is produced as both 300 kD and 500 kD complexes. Finally, BoNT/E and F are produced as only approximately 300 kD complexes. The complexes (i.e. molecular weight greater than about 150 kD) are believed to contain a non-toxin hemaglutinin protein and a non-toxin and non-toxic nonhemaglutinin protein. These two non-toxin proteins (which along with the botulinum toxin molecule comprise the relevant neurotoxin complex) may act to provide stability against denaturation to the botulinum toxin molecule and protection against digestive acids when toxin is ingested. Additionally, it is possible that the larger (greater than about 150 kD molecular weight) botulinum toxin complexes may result in a slower rate of diffusion of the botulinum toxin away from a site of intramuscular injection of a botulinum toxin complex.

*In vitro* studies have indicated that botulinum toxin inhibits potassium cation induced release of both acetylcholine and norepinephrine from primary cell cultures of brainstem tissue. Additionally, it has been reported that botulinum toxin inhibits the evoked release of both glycine and glutamate in primary cultures of spinal cord neurons and that in brain synaptosome preparations botulinum toxin inhibits the release of each of the neurotransmitters acetylcholine, dopamine, norepinephrine, CGRP and glutamate.

BoNt/A can be obtained by establishing and growing cultures of Clostridium botulinum in a fermenter and then harvesting and purifying the fermented mixture in accordance with known procedures. All the botulinum toxin serotypes are initially synthesized as inactive single chain proteins which must be cleaved or nicked by proteases to become neuroactive. The bacterial strains that make botulinum toxin serotypes A and G possess endogenous proteases and serotypes A and G can therefore be recovered from bacterial cultures in predominantly their active form. In contrast, botulinum toxin serotypes C₁, D and E are synthesized by nonproteolytic strains and are therefore typically unactivated when recovered from culture. Serotypes B and F are produced by both proteolytic and nonproteolytic strains and therefore can be recovered in either the active or inactive form. However, even the proteolytic strains that produce, for example, the BoNt/B serotype only cleave a portion of the toxin produced. The exact proportion of nicked to unnicked molecules depends on the length of incubation and the temperature of the culture. Therefore, a certain percentage of any preparation of, for example, the Bonus toxin is likely to be inactive, possibly accounting for the known significantly lower potency of BoNt/B as compared to BoNt/A. The presence of inactive botulinum toxin molecules in a clinical preparation will contribute to the overall protein load of the preparation, which has been linked to increased antigenicity, without contributing to its clinical efficacy. Additionally, it is known that BoNt/B has, upon intramuscular injection, a shorter duration of activity and is also less potent than BoNt/A at the same dose level.

It has been reported (as exemplary examples) that BoNt/A has been used clinically as follows:
(1) about 75-125 units of BOTOX®¹ per intramuscular injection (multiple muscles) to treat cervical dystonia;
   ¹Available from Allergan, Inc., of lrvine, California under the tradename BOTOX®.
(2) 5-10 units of BOTOX® per intramuscular injection to treat glabellar lines (brow furrows) (5 units injected intramuscularly into the procerus muscle and 10 units injected intramuscularly into each corrugator supercilii muscle);
(3) about 30-80 units of BOTOX® to treat constipation by intrasphincter injection of the puborectalis muscle;
(4) about 1-5 units per muscle of intramuscularly injected BOTOX® to treat blepharospasm by injecting the lateral pre-tarsal orbicularis oculi muscle of the upper lid and the lateral pre-tarsal orbicularis oculi of the lower lid.
(5) to treat strabismus, extraocular muscles have been injected intramuscularly with between about 1-5 units of BOTOX®, the amount injected varying based upon both the size of the muscle to be injected and the extent of muscle paralysis desired (i.e. amount of diopter correction desired).
(6) to treat upper limb spasticity following stroke by intramuscular injections of BOTOX® into five different upper limb flexor muscles, as follows:
   (a) flexor digitorum profundus: 7.5 U to 30 U
   (b) flexor digitorum sublimus: 7.5 U to 30 U
   (c) flexor carpi ulnaris: 10 U to 40 U
   (d) flexor carpi radialis: 15 U to 60 U
   (e) biceps brachii: 50 U to 200 U. Each of the five indicated muscles has been injected at the same treatment session, so that the patient receives from 90 U to 360 U of upper limb flexor muscle BOTOX® by intramuscular injection at each treatment session.

Additionally, it is known to inject a botulinum toxin into a urethral sphincter in order to treat urinary incontinence or urinary retention. See e.g. Wein AJ., et al., Botulinum Toxin for the Treatment of Lower Urinary Tract Symptoms: A Review and Commentary, J Urol 2005 Aug;174(2):610-2, and U.S. patent 4, 932, 936.

The tetanus neurotoxin acts mainly in the central nervous system, while botulinum neurotoxin acts at the neuromuscular junction; both act by inhibiting acetylcholine release from the axon of the affected neuron into the synapse, resulting in paralysis. The effect of intoxication on the affected neuron is long-lasting and until recently has been thought to be irreversible. The tetanus neurotoxin is known to exist in one immunologically distinct serotype.

### Acetylcholine

Typically only a single type of small molecule neurotransmitter is released by each type of neuron in the mammalian nervous system. The neurotransmitter acetylcholine is secreted by neurons in many areas of the brain, but specifically by the large pyramidal cells of the motor cortex, by several different neurons in the basal ganglia, by the motor neurons that innervate the skeletal muscles, by the preganglionic neurons of the autonomic nervous system (both sympathetic and parasympathetic), by the postganglionic neurons of the parasympathetic nervous system, and by some of the postganglionic neurons of the sympathetic nervous system. Essentially, only the postganglionic sympathetic nerve fibers to the sweat glands, the piloerector muscles and a few blood vessels are cholinergic and most of the postganglionic neurons of the sympathetic nervous system secret the neurotransmitter norepinephine. In most instances acetylcholine has an excitatory effect. However, acetylcholine is known to have inhibitory effects at some of the peripheral parasympathetic nerve endings, such as inhibition of the heart by the vagal nerve.

The efferent signals of the autonomic nervous system are transmitted to the body through either the sympathetic nervous system or the parasympathetic nervous system. The preganglionic neurons of the sympathetic nervous system extend from preganglionic sympathetic neuron cell bodies located in the intermediolateral horn of the spinal cord. The preganglionic sympathetic nerve fibers, extending from the cell body, synapse with postganglionic neurons located in either a paravertebral sympathetic ganglion or in a prevertebral ganglion. Since, the preganglionic neurons of both the sympathetic and parasympathetic nervous system are cholinergic, application of acetylcholine to the ganglia will excite both sympathetic and parasympathetic postganglionic neurons.

Acetylcholine activates two types of receptors, muscarinic and nicotinic receptors. The muscarinic receptors are found in all effector cells stimulated by the postganglionic neurons of the parasympathetic nervous system, as well as in those stimulated by the postganglionic cholinergic neurons of the sympathetic nervous system. The nicotinic receptors are found in the synapses between the preganglionic and postganglionic neurons of both the sympathetic and parasympathetic. The nicotinic receptors are also present in many membranes of skeletal muscle fibers at the neuromuscular junction.

Acetylcholine is released from cholinergic neurons when small, clear, intracellular vesicles fuse with the presynaptic neuronal cell membrane. A wide variety of non-neuronal secretory cells, such as, adrenal medulla (as well as the PC12 cell line) and pancreatic islet cells release catecholamines and insulin, respectively, from large dense-core vesicles. The PC12 cell line is a clone of rat pheochromocytoma cells extensively used as a tissue culture model for studies of sympathoadrenal development. Botulinum toxin inhibits the release of both types of compounds from both types of cells *in vitro,* permeabilized (as by electroporation) or by direct injection of the toxin into the denervated cell. Botulinum toxin is also known to block release of the neurotransmitter glutamate from cortical synaptosomes cell cultures.

As discussed above, the success rate for the treatment of a urethral stricture with the traditional techniques is not very good, due to various complications. Therefore, there exists a need for an improved method for treating a urethral stricture.

### SUMMARY

The present invention provides for methods of treating urethral strictures in a mammal, for example, in a human. In some embodiments, the methods comprise a step of administering an effective amount of a botulinum toxin directly to a urethra of the mammal, thereby treating the urethral stricture. For example, the botulinum toxin may be administered to or at the vicinity of the location of the stricture. In some embodiments, the botulinum toxin is administered to the wall of the urethra.

In some embodiments, the botulinum toxin is used in conjunction with a procedure to treat a urethra stricture. For example, the botulinum toxin may be administered in conjunction with a stenting procedure. In some embodiments, the administration of the botulinum toxin to the urethra is effective to reduce or eliminate damage to the urethra due to the stenting procedure. In some embodiments, the administration is effective to reduce or eliminate damage to the urethra by preventing the urethral epithelium from covering the stent. In some embodiments, the administration is effective to reduce or eliminate damage to the urethra by preventing the stent from becoming incorporated into the urethral wall.

The botulinum toxin may also be used in conjunction with a surgical procedure (e.g., grafting). The administration of a botulinum toxin in conjunction with these procedures may be effective to reduce or eliminate damage to the urethra due to these procedures. In some embodiments, the administration of the botulinum toxin is effective to reduce or eliminate the recurrence of the stricture, bleeding in the urethra, extravasation of irrigation fluid into perispongial tissues and/or an increase in fibrotic response.

The botulinum toxin may be any botulinum toxin including botulinum toxin type A, B, C, D, E, F, G or combinations thereof or mixtures thereof.

Any feature or combination of features described herein are included within the scope of the present invention provided that the features included in any such combination are not mutually inconsistent as will be apparent from the context, this specification, and the knowledge of one of ordinary skill in the art.

Additional advantages and aspects of the present invention are apparent in the following detailed description and claims.

### Definitions

"Agent" means as a neurotoxin, for example, a botulinum toxin, for use in accordance with the present invention. An agent can be a fragment of a neurotoxin, a modified neurotoxin or a variant neurotoxin that possesses some or all of the biological activity of an unmodified neurotoxin.

A "botulinum toxin" can be a to native botulinum toxin or a functional fragment of a botulinum toxin or a modified botulinum toxin. In addition, botulinum toxins with amino acid deletions, additions, alterations or substitutions that delete, add, alter or substitute a single amino acid, or a small percentage of amino acids (for example, less than about 5%, or for example, less than about 1%) are conservatively modified variations of botulinum toxins. Where one or more substitutions of an amino acid(s) with a chemically similar amino acid are made in a botulinum toxin, this also results in a conservatively modified variation of a botulinum toxin. Tables providing functionally similar amino acids are well known in the art. The following five groups each contain amino acids that are conservative substitutions for one another: Aliphatic: Glycine (G), Alanine (A), Valine (V), Leucine (L), Isoleucine (I); Aromatic: Phenylalanine (F), Tyrosine (Y), Tryptophan (W); Sulfur-containing: Methionine (M), Cysteine (C); Basic: Arginine (R), Lysine (K), Histidine (H); Acidic: Aspartic acid (D), Glutamic acid (E), Asparagine (N), Glutamine (Q). See also, Creighton (1984) Proteins, W.H. Freeman and Company. Conservatively modified variations of native botulinum toxins are included within the scope of the meaning of "botulinum toxin."

"Clostridial toxin" or "Clostridial neurotoxin" means a toxin produced naturally by the genus of bacteria Clostridium. For example, Clostidial toxins include, but are not limited to, botulinum toxins, tetanus toxins, difficile toxins and butyricum toxins. A Clostridial toxin can also be made by known recombinant means by a non-Clostridial bacterium.

"Combination" means an ordered sequence of elements. For example, a combination of botulinum toxins may mean administration of botulinum toxin E, followed by administration of botulinum toxin type A, followed by administration of botulinum toxin type B. This is opposed to a "mixture" where, for example, different toxin types are combined prior to administration.

"Damage" means tearing, scratching, stretching, scraping, bruising and/or inflammation or injury caused by inflammation or other injury that may occur in a urethra undergoing a procedure, for example, a procedure where the inner diameter of the urethra is expanded using mechanical force.

"Fragment" means an amino acid sequence that comprises five amino acids or more of the native amino acid sequence up to a size of minus at least one amino acid from the native sequence. For example, a fragment of a botulinum toxin type A light chain comprises five or more amino acids of the amino acid sequence of the native Botulinum toxin type A light chain up to a size of minus one amino acid from the native light chain.

"H_{C}" means a fragment obtained from the H chain of a Clostridial toxin which is equivalent, for example functionally equivalent, to the carboxyl end fragment of the H chain, or the portion corresponding to that fragment in the intact H chain involved in binding to a cell surface or cell surface receptor.

"H_{N}" means a fragment or variant obtained from an H chain of a Clostridial toxin which may be functionally equivalent to the portion of an intact H chain involved in the translocation of at least the L chain across an intracellular endosomal membrane into a cytoplasm of a cell. An H_{N}, may result from an H_{C} being removed from an H chain. An H_{N} may also result from an H chain being modified such that its H_{C} no longer binds to cholinergic cell surfaces.

"Heavy chain" means the heavy chain of a Clostridial neurotoxin or a fragment or variant of an H_{N} of a Clostridial neurotoxin. A heavy chain may have a molecular weight of about 100 kD and can be referred to as H chain, or as H.

"LH_{N}" means a fragment obtained from a Clostridial neurotoxin that contains the L chain coupled to an H_{N}. LH_{N} can be obtained from the intact Clostridial neurotoxin by proteolysis, so as to remove or to modify the H_{C} domain.

"Light chain" means the light chain of a Clostridial neurotoxin or a fragment or variant of a light chain of a Clostridial neurotoxin. A light chain may have a molecular weight of about 50 kD, and can be referred to as L chain, L, or as the proteolytic domain of a Clostridial neurotoxin.

"Linker" means a molecule which couples two or more other molecules or components together.

A "modified neurotoxin" means a neurotoxin that has a non-native component covalently attached to the neurotoxin and/or a native portion of the neurotoxin missing. For example, a modified botulinum toxin may be a light chain of a botulinum toxin with a substance P molecule covalently attached.

"Neurotoxin" or "toxin" means a substance that inhibits neuronal function or cellular secretion. Clostridial toxins are examples of a neurotoxin.

"Prevent" means to keep from occurring in whole or in part.

"Reduce" means to make smaller in magnitude (e.g. size, quantity or number). The reduction may be about 1% to about 100%. For example, the reduction may be between about 1% and about 10% or between about 10% and about 20% or between about 10% and about 30% or between about 10% and about 40% or between about 10% and about 50% or between about 10% and about 60% or between about 10% and about 70% or between about 10% and about 80% or between about 10% and about 90% or between about 10% and about 100%.

"Spacer" means a molecule or set of molecules which physically separate and/or add distance between components of agents for use in accordance with the invention.

"Substantially" means largely but not entirely. For example, substantially may mean about 10% to about 99.999%, about 20% to about 99.999%, about 30% to about 99.999%, about 40% to about 99.999% or about 50% to about 99.999%.

"Targeting component" means a molecule that has a specific binding affinity for a cell surface or cell surface receptor.

"Variant" means a molecule or peptide which is substantially the same as that of a disclosed molecule or peptide in its structure and function. For example, a variant of a specified light chain may have amino acid sequence differences when compared to the amino acid sequence of the specified light chain. Variants may be considered to be equivalent to the specifically disclosed molecules and as such are within the scope of the invention.

### DRAWINGS

Figure 1 is a diagram which shows the urinary system, including the urethra.
Figure 2a and 2b are diagrams which show the urethra in a male, and female, respectively.

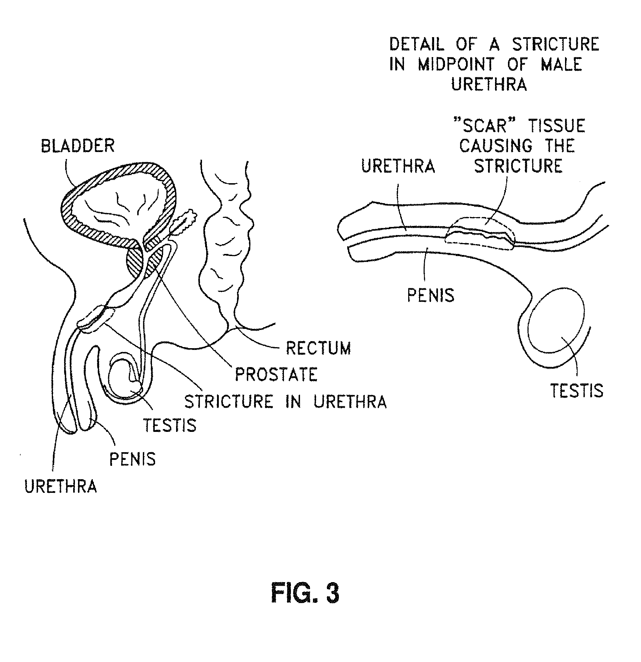

Figure 3 is a schematic diagram which shows a urethral stricture in a male patient.
Figure 4 is a diagram which shows a Urolume® stent inserted into a urethra.

### DESCRIPTION

The present invention is, in part, based upon the discovery that a neurotoxin, for example, botulinum toxin, is useful for treating urethral stricture.

In some embodiments, the methods comprise a step of administering an effective amount of a neurotoxin, e.g., a botulinum toxin, directly to a urethra of the mammal, thereby treating the urethral stricture. The neurotoxin may be administered to or at the vicinity of the location of the stricture. In some embodiments, the neurotoxin is administered to the wall of the urethra. In some embodiments, the neurotoxin is effective to reduce the stricture by more than 25%, preferably more than 50%, even more preferably more than 75%. In some embodiments, the administration of the neurotoxin, e.g., the botulinum toxin, is effective to reduce the stricture by more than 50% for more than 6 months, preferably more than 12 months, even more preferably more than two years after the administration of the neurotoxin.

As noted above, it is known to inject a botulinum toxin into a urethral sphincter in order to treat urinary incontinence or urinary retention. See e.g. Wein AJ., et al., Botulinum Toxin for the Treatment of Lower Urinary Tract Symptoms: A Review and Commentary, J Urol 2005 Aug;174(2):610-2, and U.S. patent 4, 932, 936. The urinary incontinence and urinary retention described by Wein et al. is apparently due to spasm of the urethral sphincter. Administration of the botulinum toxin to the urethral sphincter as disclosed by Wein et al. causes spastic muscle tissue in the sphincter to relax, thereby permitting voiding of urine. Significantly, a urethral stricture treatable by the present invention is not caused by a muscle spasm but is instead caused by an abnormal narrowing of the non-muscular tissue due to a buildup of fibrotic tissue, in the urethra. A buildup of fibrotic tissue can result from inflammation, ischemia, or trauma at the site of the urethra stricture. Accordingly, it is surprising that a botulinum toxin can be administered to a urethral tissue at the site of a urethral stricture, in order to treat the stricture, which stricture is not caused by a muscle contraction or by a muscle tissue spasm. Moreover, the present invention excludes administration of a botulinum toxin to a urethral sphincter as it is not the intent of the methods disclosed hereto to treat a urethral sphincter.

In some embodiments, the neurotoxin, e.g., the botulinum toxin, is used in conjunction with a procedure to treat a urethra stricture. For example, it is surprisingly discovered that a stent coated or impregnated with a botulinum may be used to effectively treat a urethral stricture. Various urethra stents are commercially available and may be used in accordance with the present invention. For example, Urolume® (sold by AMS, Minnetonka, Minnesota) is a urethral stent that may be used in accordance with the present invention. Further, these stents can be coated or impregnated with a neurotoxin, e.g., a botulinum toxin, with well known techniques (see, for example, U.S. patent 6,579,847 to Unger et al., the disclosure of which is incorporated in its entirety herein by reference).

It is noted that U.S. patent 6,579,847 to Unger et al. (filed May 1, 2000) discloses that a stent coated with a botulinum may be used to reduce a blood vessel restenosis. However, it is important to understand that a blood vessel is different from a urethra. Therefore, it is surprising that a botulinum toxin would be effective in treating a stricture in a urethra.

For example, a blood vessel is lined by a simple squamous epithelium called an endothelium. A urethra is more complex. Instead of being lined by a simple squamous epithelium, the urethra is lined by a urothelium, A urothelium is a tissue layer of approximately 3-5 cell layers and is classified as transitional epithelium. Further, the urothelium acts as a permeability barrier and protects the underlying tissues against noxious urine components. As such, one of ordinary skill would expect the urethra to be very resistant to the effects of foreign agents, e.g., a botulinum toxin. However, the invention herein surprisingly discovered that a botulinum toxin can advantageously act on the urethra to treat a stricture.

Also, drug-coated stents are currently being manufactured by Johnson & Johnson, Inc., Medtronic, Inc., Boston Scientific, Inc., and Guidant, Inc., where the stents are coated with antineoplastic agents such as Sirolimus, Sirolimus analogue ABT-578, paclitaxel, or Everolimus. Notably, these drug-coated stents are not coated with a botulinum toxin. Further, most of these drug-coated stents are used for treating a blood vessel restenosis, not a urethral stricture.

One study reports that a pacfitaxel-coated stent may be effective in treating a urethral stricture (Shin et al., Radiology 2005;234:438-444). However, a paclitaxel is very different from a botulinum toxin. For example, a paclitaxel is a small molecule and it exerts its therapeutic effects by interfering with cellular mitosis; whereas a botulinum toxin is a large protein and is not known for inhibiting cellular mitosis. Since a paclitaxel is very different from a botulinum toxin, it is surprising to discover that a botulinum toxin is effective to treat a urethral stricture.

In some embodiments, a neurotoxin, e.g., a botulinum toxin, may be administered prior to the stenting procedure in an amount of time that would allow for the urethra to dilate. In some embodiments, the neurotoxin is administered at about one week prior to the stenting procedure. Without wishing to limit the invention to any theory or mechanism of operation, it is believed that an administration of a neurotoxin, e.g., a botulinum toxin, into the urethra wall would help to dilate the urethra, provide a reduction in pain and/or help stretch the scar tissue without producing additional scars.

In some embodiments, the neurotoxin, e.g., a botulinum toxin, may be administered before, during and/or after the stenting procedure. In some embodiments a botulinum toxin type A may be administered before, during and/or after the stenting procedure.

In some embodiments, the administration of the neurotoxin, e.g., the botulinum toxin, to the urethra is effective to reduce or eliminate damage to the urethra due to the stenting procedure. In some embodiments, the administration is effective to reduce or eliminate damage to the urethra by preventing the urethral epithelium from covering the stent. In some embodiments, the administration is effective to reduce or eliminate damage to the urethra by preventing the stent from becoming incorporated into the urethral wall. It is advantageous that the urethral epithelium does not cover the stent and/or the stent does not become incorporated into the urethral wall because such event will reduce the size of the opening of the urethra which can thereby lead to a recurrent urethral stricture.

It is known to administer a botulinum toxin in combination with a urethrotomy to treat a recurrent urethral stricture. See eg Khera et al., The Journal of Urology, 2004, 172:574-575. A urethrotomy requires an incision of the urethra, which leaves wound edges. Urethral muscle contractions can act on these wound edges to cause scarring and recurrent strictures. Accordingly, Khera et al. proposes that a botulinum toxin can be administered to the urethra in combination with a urethrotomy to reduce urethral muscle contractions and prevent scarring/recurrent strictures.

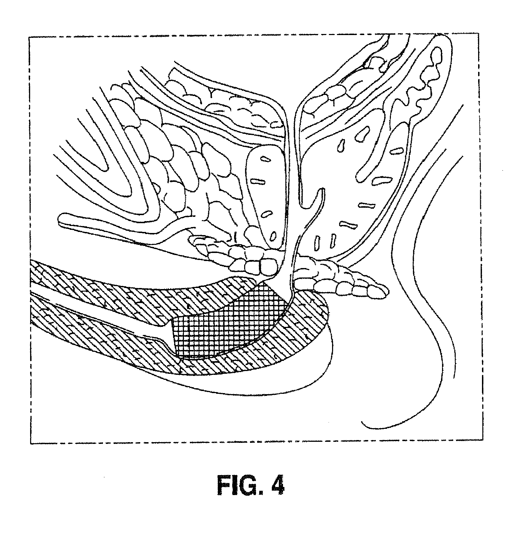

The methods disclosed herein do not involve or require any incision of the urethra. Indeed, incising the urethra or a part thereof of can be expected to be significantly counterproductive with regard to the present invention. Furthermore, in a preferred embodiment of the methods disclosed herein a stenting procedure is performed in conjunction with an administration of a botulinum toxin. A stenting procedure involves an insertion of stent (which can be coated with or embedded with a botulinum toxin) into the urethra (see eg Figure 4), and does not involve an incision of the urethra.

In some embodiments, a neurotoxin, e.g., a botulinum toxin, is administered to treat a urethra stricture in a patient, wherein the patient is not undergoing a urethrotomy.

Without wishing to limit the invention to any theory or mechanism of operation, it is believed that the toxins disclosed herein can act on inflammation mediating cells in the urothelium. These cells present many biologically active inflammation mediators which may include bradykinin and nitric oxide. Release of these and/or other mediators may contribute to the events which cause urethral inflammation which may contribute to strictures.

During secretion or exocytosis, the mediators may be included in vesicles which fuse to the inner surface of the cell membrane thereby releasing the vesicle contents to the outside of the cell. It is theorized that interference with the exocytosis process may be the mode of action of the Clostridial toxins.

It is theorized that Clostridial toxins may operate by preventing or reducing the secretion of inflammation producing molecules in the urethra cells walls or other cells by cleaving or by otherwise interfering with the function of proteins involved in the secretory process by use of a light chain component, for example, a botulinum light chain component. A heavy chain component, for example H_{N}, may also function in certain embodiments of the present invention by, for example, assisting in the release of an agent of the invention from intracellular vesicles, for example, endosomes.

Without wishing to limit the invention to any theory or mechanism of operation, it is also believed that inflammation may either directly, or indirectly contribute to strictures. By preventing or reducing urethral inflammation that may be associated with urethral procedures, for example, surgery and/or insertion of a stent, strictures may be reduced in a patient who has undergone a urethral procedure.

Another possible mechanism for the efficacy of the present disclosed invention is an effect of a botulinum toxin to inhibit neuronally mediated urethral contraction. Pretreatment with a botulinum toxin can inhibit a post stretch constriction. Within the scope of the present invention is a botulinum toxin which is a targeted toxin wherein the native binding moiety of the toxin has been replaced in whole or in part by a new binding moiety which targets the toxin to alpha2 receptors on sympathetic neurons which innervate the urethra to be treated. Furthermore, NO can be induced locally to cause dilation.

The neurotoxin for use in accordance with the present invention may comprise a targeting component, a therapeutic component and a translocation component.

In one embodiment, the targeting component comprises a carboxyl end fragment of a heavy chain of a butyricum toxin, a tetani toxin or a botulinum toxin including botulinum toxin types A, B, C, D, E, F and G.

The neurotoxin, e.g., botulinum toxin, may also be used in conjunction with a surgical procedure (e.g., grafting). For example, the administration of a botulinum toxin in conjunction with these procedures may be effective to reduce or eliminate damage to the urethra due to these procedures. In some embodiments, the administration of the botulinum toxin is effective to reduce or eliminate the recurrence of the stricture, bleeding in the urethra, extravasation of irrigation fluid into perispongial tissues and/or an increase in fibrotic response. In some embodiments, a procedure performed in conjunction with an administration of the neurotoxin, e.g., botulinum toxin, has a decrease rate of damage by more than 25%, preferably more than 50%, even more preferably more than 75% as compared to an identical procedure that is performed without the administration of a neurotoxin, e.g., a botulinum toxin. In some embodiments, a procedure performed in conjunction with an administration of a neurotoxin, e.g., the botulinum toxin is effective to reduce the stricture by more than 50% for more than 6 months, preferably more than 12 months, even more preferably more than two years after the administration of the neurotoxin.

In another embodiment the targeting component may be of non-botulinum toxin origin. Examples of targeting components that may be used in the present invention include, but are not limited to, antibodies, monoclonal antibodies, antibody fragments (Fab, F(ab)'₂, Fv, ScFv, and other antibody fragments of the like), lectins, hormones, cytokines, growth factors, peptides, carbohydrates, lipids, glycons and nucleic acids. Other targeting components that may be useful in accordance with the present invention are disclosed in WO 01/21213 which is incorporated in its entirety herein by reference.

One exemplary targeting component for use in accordance with the present invention is substance P or substances similar to substance P. Use of substance P, or substances similar to substance P, as targeting components is described in U.S. Patent Applications 09/489,667; 09/922,093 and 09/625,098 each of which is incorporated in its entirety herein by reference.

The therapeutic component operates to selectively cleave proteins essential for recognition and docking of secretory vesicles with the cytoplasmic surface of the plasma membrane, and fusion of the vesicles with the plasma membrane. One effect of the therapeutic component may be to substantially interfere with the release of neurotransmitters from a cell. Another effect of the therapeutic component may be to cause dilation of urethra. Another effect may be to cause flaccid paralysis of smooth muscle tissue. Another effect may be to reduce or eliminate secretion from cells, for example, inflammation producing cells. In one embodiment, the therapeutic component comprises a light chain of a butyricum toxin, a tetani toxin, a botulinum toxin, for example, botulinum toxin type A, B, C, D, E, F and G.

The translocation component may facilitate the transfer of at least a part of the neurotoxin, for example the therapeutic component into the cytoplasm of the target cell. In one embodiment, the translocation component comprises an amino end fragment of a heavy chain of a butyricum toxin, a tetani toxin, a botulinum toxin, for example, botulinum toxin type A, B, C, D, E, F and G.

According to a broad aspect of this invention, recombinant DNA methodologies may be used to produce the components of agents useful in accordance with the invention. These techniques may include steps of obtaining cloned genes from natural sources, or from synthetic oligonucleotide sequences, which may encode botulinum neurotoxin components including botulinum neurotoxin heavy chains, light chains or variants thereof, modified botulinum neurotoxin chains and/or fragments of the chains. Cloned genes may also encode a targeting component.

The genes may be cloned into, for example, cloning vectors, such as phages or plasmids or phagemids. The recombinant vectors are transformed into host cells, for example, into a prokaryotic cell, for example, *E. coli.* Proteins can be expressed and then isolated using conventional techniques.

Fusion genes may be used which encode more than one component of an agent. For example, a targeting component and a botulinum toxin heavy chain and/or light chain and/or a fragment of a heavy and/or a fragment of a light chain, can be produced from a single cloned gene as a fusion protein. Alternatively, individual components obtained from recombinant techniques can be chemically coupled to other components obtain from similar or other sources. For example, a targeting component may be coupled to a recombinant L chain or to a recombinant fusion LH_{N}. The linkages between the botulinum components and the targeting moieties may include appropriate spacer components, which may also be DNA encoded.

In one embodiment, an LH_{N}, which may be a hybrid of an L chain and an H_{N} from different botulinum toxin types, is expressed recombinantly as a fusion protein. Such an LH_{N} hybrid may also be coupled to a targeting component. There may be included one or more spacers between the L and H_{N} and/or between the LH_{N} and targeting component.

In another embodiment of the invention, the L chain of a botulinum neurotoxin, or a fragment of the L chain containing the endopeptidase activity, is expressed recombinantly to produce an agent for use in accordance with the present invention.

In another embodiment of the invention, the L chain of a botulinum neurotoxin, or a fragment of the L chain containing the endopeptidase activity, is expressed recombinantly as a fusion protein, with the H_{N} of the H chain and the targeting component. The expressed fusion protein may also include one or more spacer regions. For example, the L chain may be fused to H_{N} which is in turn fused to the targeting component. In another example, the H_{N} may be fused to the L chain which is in turn fused to the targeting component. Spacer components may be expressed recombinantly between some or all of the components of an agent of the invention.

In one example of producing a hybrid of LH_{N}, the L chain is obtained from botulinum toxin type B and the amine end segment of the H_{N} chain fragment is obtained from botulinum toxin type A. The H_{N} fragment of the botulinum toxin type A is produced according to the method described by Shone C. C., Hambleton, P., and Melling, J. (1987, Eur. J. Biochem. 167, 175-180) and the L chain of botulinum toxin type B according to the method of Sathyamoorthy, V. and DasGupta, B. R. (1985, J. Biol. Chem. 260, 10461-10466). The free cysteine on the amine end segment of the H chain fragment of botulinum toxin type A is then derivatized by the addition of a ten-fold molar excess of dipyridyl disulphide followed by incubation at 4°C overnight. The excess dipvridyl disulphide and the thiopyridone by product are then removed by desalting the protein over a PD10 column (Pharmacia) into PBS.

The derivatized H_{N} is then concentrated to a protein concentration in excess of 1 mg/ml before being mixed with an equimolar portion of L chain from botulinum toxin type B (>1 mg/ml in PBS). After overnight incubation at room temperature the mixture is separated by size exclusion chromatography over Superose 6 (Pharmacia), and the fractions analyzed by SDS-PAGE. The chimeric LH_{N} is then available to produce a conjugated agent which includes a targeting component.

The example described above is purely illustrative of the invention. In synthesizing the agents, the coupling of the targeting moieties to the botulinum components, for example the modified botulinum neurotoxins or fragments thereof, may be achieved via chemical coupling using reagents and techniques known to those skilled in the art. Thus, any coupling chemistry capable of covalently attaching the targeting moieties of the agents to botulinum neurotoxin components and known to those skilled in the art is covered by the scope of this application.

Modified botulinum toxins which have an altered biological persistence and/or biological activity are contemplated for use in the present invention. U.S. Patent Applications 09/620,840 and 09/910,346 include examples of compositions and methods for altering the biological persistence of botulinum toxins. These two patent applications are incorporated in their entirety herein by reference.

A biological persistence enhancing component and/or a biological activity enhancing component, for example, a leucine based motif, may be added to a botulinum neurotoxin thereby increasing the biological persistence and/or biological activity of the botulinum neurotoxin. Similarly, a biological persistence enhancing component can be removed from a botulinum neurotoxin thereby decreasing the biological persistence and/or biological activity of the neurotoxin.

The botulinum neurotoxin can be a hybrid neurotoxin. For example, the neurotoxin's targeting, translocation and therapeutic components may be derived from different botulinum toxin serotypes. For example, the polypeptide may comprise a first amino acid sequence region derived from the H_{C} of a botulinum toxin type A, a second amino acid sequence region derived from the H_{N} of botulinum type B, and a third amino acid sequence region derived from the light chain of botulinum serotype E. This is merely an example and all other possible combinations are included within the scope of the present invention.

The neurotoxin's targeting, translocation and therapeutic components can be modified from the naturally occurring sequence from which they are derived. For example, the amino acid sequence region can have at least one or more amino acids added, deleted or substituted as compared to the naturally occurring sequence.

Amino acids that can be substituted for amino acids contained in a biological persistence enhancing component include alanine, aspargine, cysteine, aspartic acid, glutamic acid, phenylalanine, glycine, histidine, isoleucine, lysine, leucine, methionine, proline, glutamine, arginine, serine, threonine, valine, tryptophan, tyrosine and other naturally occurring amino acids as well as non-standard amino acids.

Generally, the dose of neurotoxin to be administered may vary with the age, presenting condition and weight of the patient to be treated. The potency of the neurotoxin will also be considered. Toxin potency is expressed as a multiple of the LD₅₀ value for a mouse. One "unit" of toxin can be defined as the amount of toxin that kills 50% of a group of mice that were disease-free prior to inoculation with the toxin. For example, commercially available Botulinum toxin A typically has a potency such that one nanogram contains about 40 mouse units. The potency, or LD₅₀ in humans of the Botulinum toxin A product supplied by Allergan, Inc. under the registered trademark "BOTOX" is believed to be about 2,730 mouse units.

The neurotoxin can be administered in a dose of about 0.001 units up to about 100 units. In one embodiment, individual dosages of about 0.01 units to about 5 units are used. In another embodiment, individual dosages of about 0.01 units to about 3 units are used. In still another embodiment, individual dosages of about 0.01 units to about 1 unit are used. In still another embodiment, individual dosages of about 0.05 units to about 1 unit are used. Those of ordinary skill in the art will know, or can readily ascertain, how to adjust dosages for neurotoxin of greater or lesser potency in a certain circumstance.

For modified or variant botulinum toxins potency may be expressed as a multiple of the LD₅₀ value of an agent of the invention for a mouse. A "U" or "unit" of an agent can be defined as the amount of toxin that kills 50% of a group of mice that were disease-free prior to inoculation with the agent. Alternatively, potency may be expressed as the LD₅₀ value of an agent that would be produced by an equal molar amount of a native, non-variant botulinum toxin.

In an initial treatment, a low dosage may be administered to determine the patient's sensitivity to, and tolerance of, the neurotoxin. Additional administrations of the same or different dosages may be administered to the urethra as necessary. For example, a toxin may be administered to a urethra before a procedure, for example, a stenting procedure, is performed, and/or during the procedure and/or after the procedure. The number of administrations and timing of the administrations may be determined by the treating physician.

The neurotoxins may be administered by, for example, injection into a urethra using a needle or by needleless injection. The toxin may also be administered by application of the toxin to the wall of the urethra in a salve, lotion, ointment, cream, emulsion or the like delivery substrates.

An agent may be injected into the wall of a urethra from outside of the urethra or an agent may be injected into the wall of a urethra from inside the urethra. Methods for injecting into a wall of a urethra are well known to those of ordinary skill in the art. For example, an agent may be injected into the wall of a urethra from inside the urethra by the use of a catheter containing one or more needles for injecting.

In needleless injection delivery methods, microprojectile drug particles may be coated with a neurotoxin and then discharged into the urethra from an external delivery device. Depending on the discharge velocity and the distance from the injection site, the drug particles penetrate through the different layers of the urethra. As the microprojectiles penetrate through, or are deposited in, the urethra cells, the neurotoxin is released. Individual layers of urethra may be targeted for the microprojectiles.

The neurotoxins may also be administered using a stent that is coated or impregnated with the toxin, for example botulinum toxin. U.S. Patents 6,306,423 and 6,312,708 disclose material that may be impregnated, attached or imbedded with the toxin and which may be used to coat stents. In addition, the material may be used to form stents which include the toxin. The disclosure of each of these two patents is incorporated in its entirety herein by reference. In one embodiment, the urethra to be treated is first administered with botulinum toxin before inserting the toxin comprising stent. In another embodiment, the urethra to be treated is not administered with toxin before inserting the botulinum toxin comprising stent.

Administrations may be repeated if necessary. As a general guideline, botulinum toxin A administered into a urethra may produce a dilating and/or anti-inflammatory effect for, for example about 1 month to about 3 months, or for example, about 3 to about 6 months or for example from about 6 months to about 1 year.

The agent may be allowed to induce its effect on a urethra before a procedure, for example, a stenting procedure, is performed. For example, the agent may be allowed to dilate the urethra and/or prevent inflammation of the urethra before a procedure is begun. A physician of ordinary skill can determine when the agent has exerted its effect(s) on a urethra.

The invention having been fully described, examples illustrating its practice are set forth below. These examples should not, however, be considered to limit the scope of the invention, which is defined by the appended claims.

### EXAMPLES

### Example 1

### Treatment of a Urethral Stricture by Dilating the Stricture Without the Use of a Stent

Dilation is the traditional procedure for treating a urethral stricture. The dilation is customarily performed by passing a thin plastic rod (boogie) into the urethra. Rods of increasing thickness are gently inserted to gradually widen the narrowed stricture. The objective is to stretch the stricture without additional scarring. However, a stricture often tends to gradually grow narrow again after each dilation. Therefore, a repeat dilation is commonly needed.

A 35 year old male patient presents with reduced urine flow, and is diagnosed as having a urethral stricture. The patient elects to undergo a urethral dilation procedure to treat the urethral stricture. The dilation procedure is performed along with an administration of about 0.05 units and about 5 units of botulinum toxin type A (or an equivalent of the other types) to the site of the stricture on the urethra, e.g., the wall of the urethra. The administration of the botulinum toxin can be prior to, during and/or after the dilation procedure.

The urethra dilation performed in conjunction with an administration of the botulinum toxin has improved clinical results over the urethra dilation procedure performed without the administration of the botulinum toxin. For example, with the administration of the botulinum toxin, the dilation of the stricture stays dilated for a longer period of time as compared to a dilation that is not performed in conjunction with an administration of a botulinum toxin, e.g., by more than 20%, preferably more than 50%, more preferably more than 100%.

### Example 2A

### Treatment of a Urethral Stricture With a Urethral Stent

A stent is like a wire mesh tube which gives support to the urethra and helps to keep the urethra widened. A stent can be placed in the urethra by a thin instrument that is passed up into the urethra. Figure 4 shows a Urolume® stent (sold by AMS, Minnetonka, Minnesota) that is inserted into a urethra.

A 50 year old male patient presents with pain during urination. The patient is diagnosed as having a urethral stricture of 5 to 60 mm, and elects to undergo a stenting procedure. The stent is introduced under fluoroscopic guidance after internal urethrotomy or simple dilation. The stent holds the narrowed area of the urethra open for long intervals allowing for complete healing (see Figure 4).

After six months, radiologic imaging shows that the stent is covered with urethral epithelium and is incorporated into the urethral wall. The imaging also shows the urethra narrowing again at the site of the original stricture.

The patient undergoes a second stenting procedure. The second stenting procedure is performed in conjunction with an administration of about 0.05 units and about 5 units of botulinum toxin type A (or an equivalent of the other types) to the site of the stricture on the urethra, e.g., the wall of the urethra. The administration of the botulinum toxin can be prior to, during and/or after the stenting procedure.

After six months, radiologic imaging shows that the stent is not covered with urethral epithelium and is not incorporated into the urethral wall. The imaging also shows that the urethra remains adequately open at the site of the original stricture, and the patient urinates normally. After one year, radiologic imaging shows that the urethra continues to remain adequately open at the site of the original stricture, and the patient continues to urinate normally.

### Example 2B

### Treatment of a Urethral Stricture With a Urethral Stent that is Impregnated With a Botulinum Toxin

A 50 year old male patient presents with pain during urination. The patient is diagnosed as having a urethral stricture of 5 to 60 mm, and elects to undergo a stenting procedure.

The physician begins the procedure by injecting between about 0.1 units and about 5 units of botulinum toxin type A into the wall of the urethra of the patient. Following injection, the urethra is allowed to dilate. A self expanding stent impregnated with the botulinum toxin is then inserted with a catheter into the urethra. The catheter and stent are passed through the urethra to the area of stricture. A guide wire is advanced to the location of the blocked artery advancing the botulinum toxin impregnated, self expanding stent into the target area of urethra stricture. When the catheter reaches the target area, the stent is expanded bracing open the urethra.

After six months, radiologic imaging shows that the stent is not covered with urethral epithelium and is not incorporated into the urethral wall. The imaging also shows that the urethra remains adequately open at the site of the original stricture, and the patient urinates normally. After one year, radiologic imaging shows that the urethra continues to remain adequately open at the site of the original stricture, and the patient continues to urinate normally.

### Example 3

### Treatment of a Urethral Stricture with Surgery

Various surgical techniques may be used to treat a urethral stricture. For example, a short stricture can be cut out and the two ends of the healthy urethra stitched together. If the stricture is longer, then one kind of operation is similar to skin grafting the inside lining of the urethra. See Peterson et al., BJU International 2004, 94:971-976, and Barbagli et al., International Braz J Urol 2003, 29(2):155-161, the disclosures of which are incorporated in their entirety herein by reference.

A 50 year old patient with a recurring urethral stricture elects to undergo a bulbular urethroplasty procedure. Peterson et al., ld. The surgical procedure is performed in conjunction with an administration of about 0.05 units and about 5 units of botulinum toxin type A (or an equivalent of the other types) to the site of the stricture on the urethra, e.g., the wall of the healthy urethra and/or the graft tissue. The botulinum toxin may be administered before, during and/or after the surgical procedure.

Two years after the procedure, there is no sign stricture reformation, and the patient appears to be in good health.

### Example 4

### Treatment of a Urethral Stricture With a Urethral Stent that is Impregnated With a Botulinum Toxin

A 34 year old female patient presents with difficulty urinating. The patient is diagnosed as having a urethral stricture and agrees to placement of a botulinum toxin coated (or impregnated) stent.

A self expanding stent coated impregnated with the botulinum toxin is inserted with a catheter into the urethra. The catheter and stent are passed through the urethra to the area of the stricture. A guide wire is advanced to the location of the blocked artery advancing the botulinum toxin impregnated, self expanding stent into the target area of urethra stricture. When the catheter reaches the target area, the stent is expanded bracing open the urethra. The stent can be removable or can remain in place. The stent can be made of a non-biodegradable material (such as a polyvinyl polymer) or the stent can be made of a biodegradable polymer such as a polylactic polyglycolic acid copolymer (PLGA). See eg. U.S. patents 6,306,423; 6,312,708; 6,506,399; 6,383,509, and; 6,585,993.

After six months, radiologic imaging shows that the stent is not covered with urethral epithelium and is not incorporated into the urethral wall. The imaging also shows that the urethra remains adequately open at the site of the original stricture, and the patient urinates normally. After one year, radiologic imaging shows that the urethra continues to remain adequately open at the site of the original stricture, and the patient continues to urinate normally.

My invention also includes within its scope use of a botulinum toxin in the preparation of a medicament (i.e. a botulinum toxin coated stent) for treatment of a urethral stricture.

All references, articles, patents, applications and publications set forth above are incorporated herein by reference in their entireties.

Accordingly, the spirit and scope of the following claims should not be limited to the descriptions of the preferred embodiments set forth above.

The present invention can be summarized by reference to the following embodiments (embs.) :
emb. 1. A method for treating a urethral stricture in a patient, the method comprising the step of administering an effective amount of a botulinum toxin directly to a urethra of the patient, wherein the patient is having or has had a stenting procedure, thereby treating the urethral stricture.
emb. 2. The method of emb. 1 wherein the step of administering includes injecting the botulinum toxin into a wall of the urethra.
emb. 3. The method of emb. 1 wherein the step of administering is accomplished using a stent coated or impregnated with the botulinum toxin.
emb. 4. The method of emb. 1 wherein the botulinum toxin reduces or eliminates a damage to the urethra that is due to the stenting procedure.
emb. 5. The method of emb. 1 wherein the botulinum toxin reduces or eliminates a damage to the urethra by reducing the recurrence of the stricture.
emb. 6. The method of emb. 1 wherein the botulinum toxin reduces or eliminates a damage to the urethra by preventing the urethral epithelium from covering the stent.
emb. 7. The method of emb. 1 wherein the botulinum toxin reduces or eliminates a damage to the urethra by preventing the stent from becoming incorporated into the urethral wall.
emb. 8. The method of emb. 1 wherein the botulinum toxin reduces or eliminates a damage to the urethra by reducing or eliminating bleeding in the urethra.
emb. 9. The method of emb. 1 wherein the botulinum toxin reduces or eliminates damage to the urethra by reducing or eliminating extravasation of irrigation fluid into perispongial tissues.
emb. 10. The method of emb. 1 wherein the botulinum toxin reduces or eliminates damage to the urethra by preventing an increase in fibrotic response.
emb. 11. The method of emb. 1 wherein the botulinum toxin is selected from the group consisting of botulinum toxin types A, B, C, D, E, F, G and combinations thereof.
emb. 12. The method of emb. 1 wherein the botulinum toxin is botulinum toxin type A.
emb. 13. A method for treating a urethral stricture in a mammal, the method comprising the step of
   (a) administering an effective amount of a botulinum toxin directly to a urethra of the mammal; and
   (b) stenting the urethra.
14. The method of emb. 13 wherein the administering includes the step of injecting the botulinum toxin into a wall of the urethra.
emb. 15. The method of emb. 13 wherein the step of administering is accomplished using a stent coated or impregnated with the botulinum toxin.
emb. 16. The method of emb. 13 wherein the botulinum toxin is selected from the group consisting of botulinum toxin types A, B, C, D, E, F, G and combinations thereof.
emb. 17. The method of emb. 13 wherein the botulinum toxin is botulinum toxin type A.
emb. 18. A method for treating a urethral stricture in a patient, the method comprising the step of administering an effective amount of a botulinum toxin directly to a urethra of the patient, wherein the patient is not having a urethrotomy, thereby treating the urethral stricture.
emb. 19. The method of emb. 18 wherein the botulinum toxin is selected from the group consisting of botulinum toxin types A, B, C, D, E, F, G and combinations thereof.
emb. 20. The method of emb. 18 wherein the botulinum toxin is botulinum toxin type A.
emb. 21. A method for treating a urethral stricture, the method comprising the step of inserting into a urethral stricture of a patient a biodegradable stent coated with or embedded with a therapeutically effective amount of a botulinum toxin, thereby treating the urethral stricture.

## Claims

1. A botulinum toxin for use in a method of treating a urethral stricture in a patient, the method comprising the step of administering an effective amount of a botulinum toxin directly to a urethra of the patient, wherein the step of administering is accomplished using a stent coated or impregnated with the botulinum toxin, wherein the botulinum toxin reduces or eliminates a damage to the urethra that is due to the stenting procedure.

2. The botulinum toxin for use of claim 1, wherein the botulinum toxin reduces or eliminates a damage to the urethra by reducing the recurrence of the stricture.

3. The botulinum toxin for use of claim 1, wherein the botulinum toxin reduces or eliminates a damage to the urethra by preventing the urethral epithelium from covering the stent or by preventing the stent from becoming incorporated into the urethral wall.

4. The botulinum toxin for use of claim 1, wherein the botulinum toxin reduces or eliminates a damage to the urethra by reducing or eliminating bleeding in the urethra or by reducing or eliminating extravasation of irrigation fluid into perispongial tissues or by preventing an increase in fibrotic response.

5. The botulinum toxin for use of claim 1, wherein the botulinum toxin is selected from the group consisting of botulinum toxin types A, B, C, D, E, F, G and combinations thereof.

6. The botulinum toxin for use of claim 1, wherein the botulinum toxin is botulinum toxin type A.

7. A botulinum toxin for use in a method of treating a urethral stricture in a mammal, the method comprising the step of
(a) administering an effective amount of a botulinum toxin directly to a urethra of the mammal; and
(b) stenting the urethra.

8. The botulinum toxin for use of claim 7, wherein the administering includes the step of injecting the botulinum toxin into a wall of the urethra.

9. The botulinum toxin for use of claim 7, wherein the step of administering is accomplished using a stent coated or impregnated with the botulinum toxin.

10. The botulinum toxin for use of claim 7, wherein the botulinum toxin is selected from the group consisting of botulinum toxin types A, B, C, D, E, F, G and combinations thereof.

11. The botulinum toxin for use of claim 7, wherein the botulinum toxin is botulinum toxin type A.

12. A botulinum toxin for use in a method of treating a urethral stricture in a patient, the method comprising the step of administering an effective amount of a botulinum toxin directly to a urethra of the patient, wherein the patient is not having a urethrotomy, thereby treating the urethral stricture.

13. The botulinum toxin for use of claim 12, wherein the botulinum toxin is selected from the group consisting of botulinum toxin types A, B, C, D, E, F, G and combinations thereof.

14. The method of claim 12, wherein the botulinum toxin is botulinum toxin type A.

15. A botulinum toxin for use in a method of treating a urethral stricture, the method comprising the step of inserting into a urethral stricture of a patient a biodegradable stent coated with or embedded with a therapeutically effective amount of a botulinum toxin, thereby treating the urethral stricture.
